Europäisches Patentamt

European Patent Office

Office européen des brevets

(11) Numéro de publication: **0 350 371**
**A1**

# (12) DEMANDE DE BREVET EUROPEEN

(21) Numéro de dépôt: **89401844.9**

(22) Date de dépôt: **28.06.89**

(51) Int. Cl.⁵: **A 61 F 2/42**

(30) Priorité: **07.07.88 FR 8809203**

(43) Date de publication de la demande:
**10.01.90 Bulletin 90/02**

(84) Etats contractants désignés:
**BE CH DE ES GB IT LI SE**

(71) Demandeur: **Lesur, Etienne**
**12, Boulevard Albert 1er**
**F-54000 Nancy (FR)**

(72) Inventeur: **Lesur, Etienne**
**12, Boulevard Albert 1er**
**F-54000 Nancy (FR)**

(74) Mandataire: **Flavenot, Bernard**
**Société ABRITT 17, rue du Docteur Charcot La Norville**
**F-91290 Arpajon (FR)**

(54) **Pièce de prothèse pour poignet.**

(57) La présente invention concerne les pièces de prothèses pour poignet, notamment en remplacement de l'os semi-lunaire et d'une partie de l'os scaphoïde, en vue de leur articulation par rapport aux autres os du poignet et notamment par rapport au grand os.

La pièce selon l'invention se caractérise essentiellement par le fait qu'elle comporte un élément solide (2) ayant une surface latérale (3) torique de révolution et délimité à ses deux extrémités par une première (6) et une seconde (7) faces, la première face d'extrémité (6) étant définie dans un premier plan (17) contenant l'axe de révolution (9), la seconde face d'extrémité (7) étant définie dans un second plan faisant, d'une part, un angle sensiblement de trente degrés par rapport à un rayon de révolution (11) de la surface torique et, d'autre part, un angle sensiblement de trente degrés par rapport à l'axe de révolution, l'élément comportant en outre une cavité (13) sensiblement hémisphérique ayant une ouverture (14) centrée sensiblement sur la génératrice de plus faible rayon de révolution.

fig. 1

EP 0 350 371 A1

Description

## Pièce de prothèse pour poignet

La présente invention concerne une pièce de prothèse pour poignet, plus particulièrement une pièce permettant de remplacer tout ou partie de certains os du poignet, par exemple le scaphoïde et le semi-lunaire qui sont des os très souvent sujets à des détériorations causées par des accidents ou des maladies.

Chaque individu possède un outil très important qui lui permet de réaliser nombre de mouvements nécessaires à la vie de tous les jours. Cet outil est la main. Cette main est rattachée aux os de l'avant-bras, le radius et le cubitus, par le poignet dont la structure ostéoligamentaire complexe comprend notamment trois os principaux : le scaphoïde, le semi-lunaire et la grand os.

Malheureusement, ces os et certains des ligaments qui les unissent peuvent subir des détériorations mécaniques dues, par exemple, à des traumatismes tels que la rupture des ligaments scapho-lunaires ou une fracture du scaphoïde, ou à des maladies comme la nécrose, notamment du semi-lunaire.

Dans de tels cas, le practicien peut être obligé de procéder à l'implantation d'une prothèse pour remplacer certains des os du poignet, en particulier le scaphoïde et le semi-lunaire, et pour pallier de graves lésions ligamentaires. Les prothèses utilisées jusqu'à aujourd'hui sont constituées d'une pièce unique remplaçant l'un ou l'ensemble de ces deux os. Bien que permettant théoriquement de redonner au poignet une indolence, une stabilité et une amplitude angulaire essentielles pour orienter correctement la main, du moins pour les mouvements principaux, cette technique présente malgré tout les inconvénients d'éliminer les déplacements utiles pour les mouvements, existant naturellement entre les faces en contact de ces deux os essentiels, et de ne pas présenter les points d'ancrage corrects pour les ligaments attachés, notamment, au tubercule pour le scaphoïde et aux cornes pour le semi-lunaire.

Ce type de prothèse limite donc les possibilités de restitution du déplacement physiologique de l'ensemble du poignet par rapport à l'avant-bras.

La présente invention a pour but de réaliser une pièce de prothèse en remplacement de certains os du poignet, qui permette au poignet de conserver une stabilité qui n'était pas possible avec les prothèses selon l'art antérieur, qui soit d'un réalisation industrielle des plus simple, par exemple par la technique des robots d'usinage, et qui soit d'une implantation dans le poignet relativement facile, tout en s'articulant parfaitement avec les autres os du poignet conservés en l'état ou remplacés.

Plus précisément, la présente invention a pour objet une pièce de prothèse pour poignet, notamment en remplacement de l'os semi-lunaire et d'une partie de l'os scaphoïde, en vue de leur articulation par rapport aux autres os du poignet et notamment par rapport au grand os, caractérisée par le fait qu'elle comporte un élément ayant une surface latérale définie dans une forme torique de révolution et délimité à ses deux extrémités par une première et une seconde faces d'extrémité, ladite surface torique étant définie par un centre, un axe de révolution passant par ledit centre, et des rayons de révolution perpendiculaires à l'axe de révolution et passant par ledit centre,

la première face d'extrémité étant définie dans un premier plan contenant ledit axe de révolution,

la seconde face d'extrémité étant définie dans un second plan faisant, d'une part, un angle sensiblement de trente degrés par rapport à un rayon de révolution de la surface torique et, d'autre part, un angle sensiblement de trente degrés par rapport audit axe de révolution,

ledit élément comportant en outre une cavité sensiblement hémisphérique ayant une ouverture sensiblement centrée sur la génératrice de plus faible rayon de révolution de ladite surface torique et dans le plan bissecteur de deux plans contenant ledit axe de révolution et passant respectivement par les deux points appartenant respectivement aux deux dites face d'extrémité et à la génératrice de plus grand rayon de révolution.

D'autres caractéristiques et avantages de la présente invention apparaîtront au cours de la description suivante donnée en regard des dessins annexés à titre illustratif, mais nullement limitatif, dans lesquels :

La figure 1 représente une vue de côté d'une pièce de prothèse selon l'invention,

La figure 2 représente une coupe de la pièce selon la figure 1, référencée II-II sur cette figure, permettant de bien faire comprendre sa forme structurelle dans le cadre de sa fonction selon l'invention, et

La figure 3 représente une coupe frontale d'une main droite, montrant l'implantation de la pièce selon l'invention en association avec les os du poignet et de la main, cette dernière y étant vue du côté de la paume.

Les trois figures représentant un même mode de réalisation d'une pièce de prothèse pour poignet selon l'invention, les mêmes références y désignent les mêmes éléments, quelles que soient les figures sur lesquelles elles apparaissent.

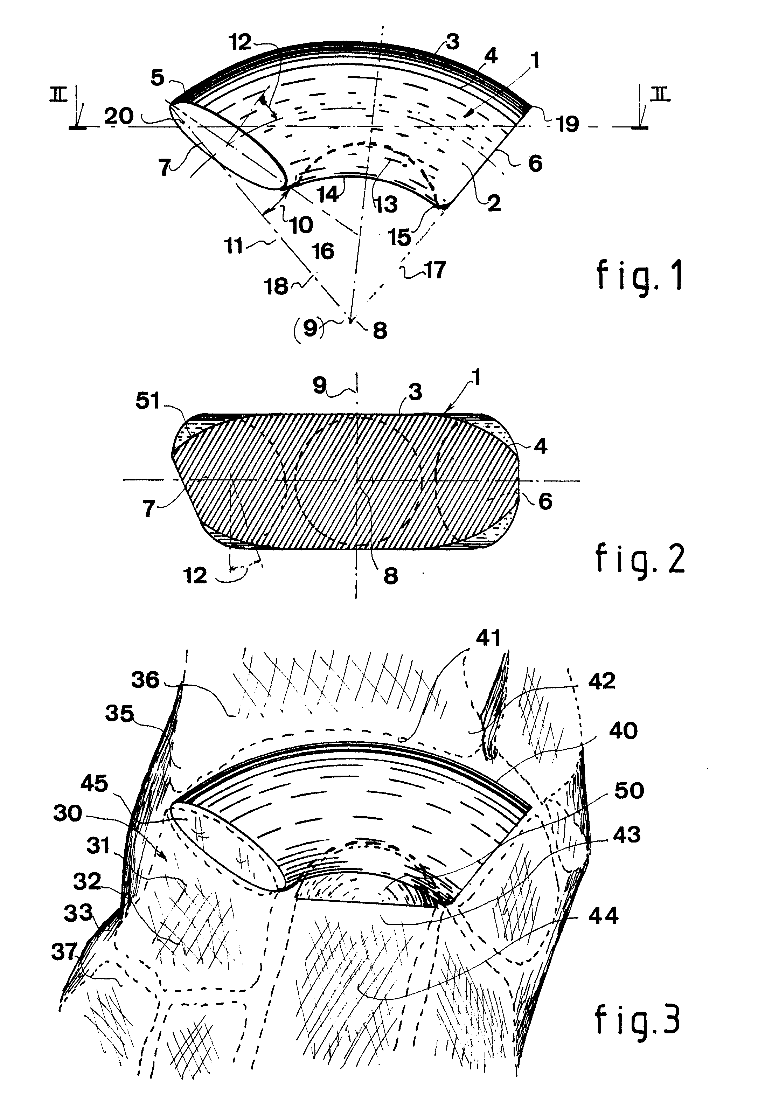

En considérant plus particulièrement les figures 1 et 2, celles-ci représentent un mode de réalisation d'une pièce de prothèse 1 pour poignet en accord avec l'invention. Cette pièce 1 est réalisée en un matériau solide, par exemple du titane, un alliage inoxydable ou de la céramique. Elle est conçue pour remplacer une partie de l'os scaphoïde et l'os semi-lunaire dans son entier, en vue de leur articulation par rapport aux autres os du poignet, notamment par rapport au grand os.

Cette pièce comporte un élément 2 ayant une surface latérale 3 définie dans une forme torique de révolution et délimité à ses deux extrémités 4, 5 par une première 6 et une seconde 7 faces. La surface torique est donc définie de façon classique par un centre 8, un axe de révolution 9 passant par le centre 8, et des rayons de révolution perpendiculaires à l'axe de révolution et passant par le centre.

La première face 6 est définie dans un plan contenant l'axe de révolution 9 et donc perpendiculaire aux génératrices de la surface torique 3.

La seconde face 7 est définie dans un deuxième plan faisant, d'une part, un angle 10 sensiblement de trente degrés par rapport à un rayon de révolution 11 de la surface torique 3 et, d'autre part, un angle 12 sensiblement de trente degrés par rapport à l'axe de révolution 9. Cet angle 12 n'a seulement été qu'évoqué sur les deux figures 1 et 2, étant donné qu'il est difficile de le représenter.

L'élément 2 comporte en outre une cavité 13 sensiblement hémisphérique ayant une ouverture 14 sensiblement centrée sur la génératrice 15 de plus faible rayon de révolution de la surface torique, le centre de cette cavité étant cependant avantageusement située en dehors de la surface torique pour que la profondeur de la cavité soit inférieure à son rayon, de façon qu'une sphère complémentaire puisse y être facilement introduite. Le centre de cette cavité 13 est de plus situé dans le plan bissecteur 16 des deux plans 17, 18 contenant l'axe de révolution 9 et passant respectivement par les deux points 19, 20 appartenant respectivement aux deux faces d'extrémité 6, 7 et à la génératrice de plus grand rayon de révolution.

Dans un mode avantageux de réalisation, l'angle que font les deux plans 17, 18 est sensiblement de l'ordre de quatre-vingts degrés, cette valeur d'angle permettant de répondre à presque tous les cas de prothèse pour le poignet telle que définie ci-dessus, et le plan de la seconde face d'extrémité 7 coupe le plan bissecteur 16 suivant une droite située entre l'axe de révolution 9 et l'élément 1.

Avec une telle pièce de prothèse pour poignet, il est donc possible de remplacer, comme représenté sur la figure 3, l'os semi-lunaire et la partie proximale du scaphoïde 30 qui est lié avec le semi-lunaire par le ligament radio-scapho-lunaire, tout en conservant la partie distale 31 du scaphoïde, c'est-à-dire son tubercule 32 qui est le siège de l'insertion d'importants ligaments, par exemple les ligaments scapho-trapéziens 33 et le ligament - non représenté sur la figure 3 reliant la styloïde radiale 35 du radius 36 au grand os 44 via le scaphoïde 30, pour conserver la commande, par exemple, du déplacement du complexe scapholunaire.

Pour cela, le scaphoïde est coupé de façon à éliminer sa partie proximale, mais à laisser en place se partie distale 31, dont le tubercule 32, et ainsi conserver l'ensemble des ligaments qui y sont insérés.

La pièce de prothèse telle que définie ci-dessus est disposée dans le poignet de façon que sa courbure 40 la plus grande soit positionnée essentiellement contre la surface articulaire 41 de l'extrémité distale 42 du radius 36 et que la cavité hémisphérique 13 épouse la tête sphérique 43 du grand os 44 du poignet qui collabore naturellement et normalement avec une partie en creux complémentaire dans le semi-lunaire et dans le scaphoïde.

Ainsi disposée, cette pièce de prothèse 1 remplace le semi-lunaire et la partie proximale du scaphoïde dont la face de coupe 45 s'applique parfaitement contre la deuxième face d'extrémité 7 de la prothèse 1, sans nécessiter d'éléments de liaison complémentaires, l'inclinaison de cette face de coupe et celle de la seconde face d'extrémité 7 étant déterminées pour que ces faces soient sensiblement perpendiculaires à la résultante de l'ensemble des forces exercées. Cette conformation apporte à la prothèse la qualité indispensable pour une bonne mobilité et une stabilité fiable : l'autostabilisation.

En effet, la pente de la deuxième face d'extrémité 7 de la prothèse et celle de la face de coupe 45 du scaphoïde, ainsi que les forces de liaison exercées par les ligaments restant insérés sur le tubercule du scaphoïde, permettent de bien maintenir en coopération la partie restante du scaphoïde et la pièce de prothèse. Cette liaison par simple application maintient bien l'ensemble des os du poignet les uns par rapport aux autres, et permet de conserver les mouvements principaux du poignet, ce que les prothèses antérieures ne permettaient pas, notamment dans les mouvements d'inclinaison radiale et cubitale selon un axe normal au plan dans lequel se situe la main. Le déplacement relatif de la pièce de prothèse 1 et de la partie distale restante 32 du scaphoïde dans le plan de leurs deux faces en contact permet lui-même d'obtenir certains mouvements avec plus de souplesse que dans le cas des réalisations de l'art antérieur connues.

Bien évidemment, il arrive aussi que la partie sphérique du grand os 44 ait subi des détériorations, en même temps que le semi-lunaire. Dans ce cas, cette partie est elle aussi éliminée et remplacée par une portion de sphère 50 solidarisée au grand os 44, par exemple au moyen d'un pivot ou d'une vis. Cette portion de sphère 50 constitue la partie mâle d'un rotule dont la partie femelle est constituée par la cavité hémisphérique 13 réalisée dans la pièce de prothèse 1, comme décrit ci-avant.

Aux avantages de cette pièce de prothèse mentionnés ci-dessus, il faut ajouter celui de sa réalisation mécanique industrielle des plus aisé, les différentes formes entrant dans sa structure, de révolution ou planes, permettant un usinage par robots.

De plus, cette pièce de prothèse peut être très facilement implantée dans le poignet, car cette implantation ne nécessite pas d'éléments de liaison complémentaires, simplement une intervention de coupe plane d'un os.

## Revendications

1. Pièce de prothèse pour poignet, notamment en remplacement de l'os semi-lunaire et d'une partie de l'os scaphoïde, en vue de leur articulation par rapport aux autres os du poignet et notamment par rapport au grand os (44), caractérisée par le fait qu'elle comporte un élément (2) ayant une surface latérale (3) définie dans une forme torique de révolution et délimité à ses deux extrémités par une première (6) et une seconde (7) faces d'extrémité, ladite surface torique étant définie par un centre (8), un axe de révolution (9) passant par ledit centre, et des rayons de révolution (11) perpendiculaires à l'axe de révolution et passant par ledit centre,

la première face d'extrémité (6) étant définie dans un premier plan (17) contenant ledit axe de révolution,

la seconde face d'extrémité (7) étant définie dans un second plan faisant, d'une part, un angle (10) sensiblement de trente degrés par rapport à un rayon de révolution (11) de la surface torique et, d'autre part, un angle (12) sensiblement de trente degrés par rapport audit axe de révolution (9),

ledit élément (2) comportant en outre une cavité (13) sensiblement hémisphérique ayant un ouverture (14) sensiblement centrée sur la génératrice de plus faible rayon de révolution de ladite surface torique et dans le plan bissecteur (16) des deux plans (17,18) contenant ledit axe de révolution (9) et passant respectivement par les deux points (19,20) appartenant respectivement aux deux dites faces d'extrémité (6,7) et à la génératrice de plus grand rayon de révolution.

2. Pièce de prothèse selon la revendication 1, caractérisée par le fait que l'angle que font les deux plans (17,18) contenant l'axe de révolution (9) et passant par les deux points (19,20) appartenant respectivement aux deux faces d'extrémité (6,7) et à la génératrice de plus grand rayon de révolution est de l'ordre de quatre-vingts degrés.

3. Pièce de prothèse selon l'une des revendications précédentes, caractérisée par le fait que ladite cavité hémisphérique (13) constitue la partie femelle d'une rotule dont la partie mâle complémentaire est constituée par une portion de sphère (50) apte à être implantée sur le grand os (44).

4. Pièce de prothèse selon l'une des revendications précédentes, caractérisée par le fait que la profondeur de ladite cavité hémisphérique (13) est inférieure à la valeur de son rayon.

5. Pièce de prothèse selon l'une des revendications précédentes, caractérisée par le fait que ledit élément (2) est réalisé en un matériau solide, par exemple du titane.

6. Pièce de prothèse selon l'une des revendications précédentes, caractérisée par le fait que le plan de ladite seconde face d'extrémité (7) coupe ledit plan bissecteur (16) suivant une droite située entre ledit axe de révolution (9) et ledit élément (1).

fig.1

fig.2

fig.3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,P | US-A-4 784 661  (BECKENBAUGH et al.) <br> * Résumé; figures 7,8 * <br> --- | 1 | A 61 F   2/42 |
| A | EP-A-0 034 192  (HOWMEDICA INTERNATIONAL INC.) <br> * Figure 1,6 * <br> --- | 1 | |
| A | DE-A-2 424 537  (NATIONAL RESEARCH DEVELOPMENT CORP.) <br> * Figures 1,2 * <br> ----- | 1 | |

**DOMAINES TECHNIQUES RECHERCHES (Int. Cl.5)**

A 61 F

Le présent rapport a été établi pour toutes les revendications

| Lieu de la recherche | Date d'achèvement de la recherche | Examinateur |
|---|---|---|
| LA HAYE | 10-10-1989 | ARGENTINI A. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
........................................................................................
& : membre de la même famille, document correspondant

EPO FORM 1503 03.82 (P0402)